# EUROPEAN PATENT APPLICATION

(11) **EP 3 753 581 A1**
(43) Date of publication of application: **23.12.2020**
(21) Application number: 19739186.5
(22) Date of filing: 29.04.2019
(51) Int. Cl.: A61L 2/10, A61L 2/26, A61L 2/24

(54) **IDENTIFICATION METHOD, CONSTANT-TEMPERATURE ASEPTIC PRESERVATION METHOD AND STERILIZATION CONTAINER**

(30) Priority: 11.04.2019 CN 201910290981; 11.04.2019 CN 201920489067 U
(71) Applicant: Shenzhen Uvled Optical Technology Co., Ltd., Shenzhen City, Guangdong 518000 (CN)
(72) Inventor: OU YANG, CHENYI, YONGZHOU CITY, Hunan 425600 (CN)
(74) Representative: Viering, Jentschura & Partner mbB Patent- und Rechtsanwälte
(86) International application number: PCT/CN2019/085047
(87) International publication number: WO 2020/206767

(57) **Abstract**

The invention discloses an identification method, a constant temperature aseptic storage method and a sterilization container, and relates to the technical field of sterilization. The identification method maintains the sterilized article at a preset constant temperature after the sterilization is finished, and utilizes the temperature of the sterilized article for identifying whether the sterilized article is sterilized. The constant temperature aseptic storage method is to control the illumination device and the heating air duct of the sterilization container to run in a low-power operation, and continuously release a small amount of ultraviolet light and a small amount of hot air to the article storage cavity of the sterilization container so as to maintain the sterilized article at a preset constant temperature and aseptic condition. The sterilization container is capable of performing the identification method and the constant temperature sterilization method.

## Description

### BACKGROUND

### 1. Technical Field

The invention relates to the field of sterilization technology, in particular to an identification method applied to a sterilization container for identifying whether a sterilized article is sterilized, a constant temperature aseptic storage method applied to a sterilization container, and a sterilization container.

### 2. Description of Related Art

With the improvement of living standards, people gradually pay attention to the quality of life. Sterilization containers used to sterilize various household items, such as tableware, baby products, etc., have become essential appliances for people. According to the sterilization method, the common ones are ultraviolet and ozone.

These sterilization containers are basically effective in killing bacteria on the sterilized article for sterilization purposes. However, there are still some shortcomings. 1. There is no identification function. Tableware, baby products and other daily necessities are often not used immediately after sterilization, and people's operations will always be negligent. If you forget to start the sterilization container, you can not find it when using these daily necessities. 2. The user experience is not good. In places with low temperatures, people, especially babies, feel uncomfortable when using these household items because of the cold environment. 3. It is easy to be contaminated again before use. Since it is not used immediately after sterilization, residual bacteria, or bacteria in the environment, can reproduce and contaminate these household items.

### SUMMARY

The first object of the present invention is to provide an identification method to assist a user in identifying whether a sterilized article is sterilized.

The second object of the present invention is to provide a constant temperature aseptic storage method for achieving constant temperature and aseptic storage using the original structure of the sterilization container, giving the user a better experience and avoiding contamination of the sterilized article before use.

The third object of the present invention is to provide a sterilization container that can assist a user in identifying whether a sterilized article is sterilized, and/or can achieve constant temperature aseptic storage using the original structure of the sterilization container.

In order to achieve the above first object, the present invention provides an identification method applied to a sterilization container for identifying whether a sterilized article is sterilized, wherein the identification method comprises the following steps: controlling a heating air duct of the sterilization container to run in a low-power operation after the sterilization is finished; and continuously releasing a small amount of hot air to an article storage cavity of the sterilization container to maintain the sterilized article in the article storage cavity at a preset constant temperature; wherein the constant temperature is defined as when the sterilized article is in contact with the human body, the human body feels warm and not uncomfortable.

Preferably, the sterilization container is preset with a plurality of different constant temperatures; and in the identification method, the sterilization container acquires an ambient temperature and adaptively selects an appropriate constant temperature according to the ambient temperature.

Preferably, in the identification method, the method for obtaining the ambient temperature of the sterilization container is to use the intracavity temperature estimation of the article storage cavity.

In order to achieve the above second object, the present invention provides a constant temperature aseptic storage method for a sterilization container, wherein the constant temperature aseptic storage method comprises: after issuing a constant temperature aseptic storage instruction, the sterilization container performing the following steps: controlling a heating air duct and an illumination device of the sterilization container to run in a low-power operation, and continuously releasing a small amount of ultraviolet light and a small amount of hot air to an article storage cavity of the sterilization container to maintain a sterilized article in the article storage cavity at a preset constant temperature and an aseptic condition; wherein the constant temperature is defined as when the sterilized article is in contact with the human body, the human body feels warm and not uncomfortable.

Preferably, the sterilization container is preset with a plurality of different constant temperatures, and in the constant temperature aseptic storage method, the sterilization container acquires an ambient temperature and adaptively selects an appropriate constant temperature according to the ambient temperature.

Preferably, the method for obtaining the ambient temperature of the sterilization container is to use the intracavity temperature estimation of the article storage cavity.

In order to achieve the above third object, the present invention provides a sterilization container, comprising: an article storage cavity for accommodating a sterilized article; an illumination device for releasing a sterilization medium to the article storage cavity; a heating air duct for releasing hot air to the article storage cavity; a microprocessor electrically coupled to the illumination device and the heating air duct, respectively; a memory storing a computer readable program executable by the microprocessor; wherein the sterilization container performs the steps of the aforesaid identification method and/or the steps of the aforesaid constant temperature aseptic storage when the computer readable program is executed by the microprocessor.

Preferably, the sterilization container is a portable ultraviolet sterilization container.

Preferably, the heating air duct is an inner circulation type heating air duct.

Preferably, an electric heating element of the heating air duct comprises a resistance wire and a frame having a sheet shape and a hollow portion; wherein the resistance wire is wound around the frame and a mesh structure is formed in the hollow portion.

Compared with the prior art, the present invention has at least the following beneficial effects.

The identification method, which uses temperature to identify whether a sterilized article is sterilized, can assist the user to accurately identify whether a sterilized article is sterilized. In addition, by virtue of the present invention, when the sterilized article is used, the temperature can give a comfortable experience. Especially in cold environments, this comfortable experience is more remarkable.

The present invention creatively proposes to realize a constant temperature aseptic storage in the sterilization container, which not only can effectively prevent the sterilized article from being re-contaminated before use, but also has a warm feeling when the sterilized article is used at any time. It can give users a comfortable experience, especially in cold environments, and this comfortable experience is more remarkable. In addition, the constant temperature aseptic storage method is realized by using the original structure of the sterilization container, and the cost is low.

### BRIEF DESCRIPTION OF THE DRAWINGS

FIG. 1 is a flow chart of the identification method.
FIG. 2 is a flow chart of the constant temperature aseptic storage method.
FIG.3 is a schematic structural view of the article storage cavity, the illumination device, and the heating air duct of the sterilization container.
FIG. 4 is a schematic view of the fan and the air duct in the heating air duct.
FIG. 5 is a schematic structural view of the electric heating element.

Reference numerals: 1, article storage cavity; 2, return air outlet; 3, air outlet; 4, sterilization container body; 5, handle; 6, illumination device (UV LED); 7, air duct; 7', fan; 8, electric heating elements; 9, frame; 10, resistance wire; 11, hollow portion.

### DESCRIPTION OF THE EXEMPLARY EMBODIMENTS

The invention will be further described below in conjunction with the drawings and embodiments.

This identification method is applied to a sterilization container for identifying whether a sterilized article is sterilized.

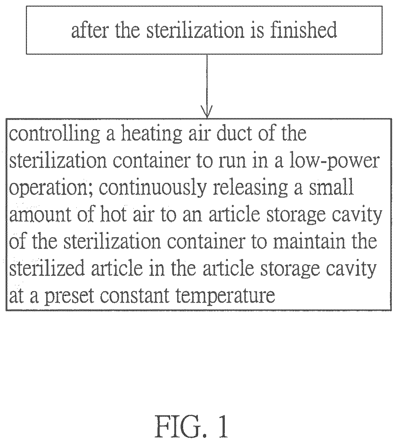

Referring to FIG. 1, the identification method includes the following steps: controlling a heating air duct of the sterilization container to run in a low-power operation after the sterilization is finished; and continuously releasing a small amount of hot air to an article storage cavity of the sterilization container to maintain the sterilized article in the article storage cavity at a preset constant temperature; wherein the constant temperature is defined as when the sterilized article is in contact with the human body, the human body feels warm and not uncomfortable.

When the user takes out the sterilized article from the sterilization container, the warm feeling can be felt, that is, the sterilized article has been sterilized, and if there is no warm feeling, it means that the sterilized article has not been sterilized. This prevents people from inadvertently forgetting to start the sterilization container and using unsterilized items.

The identification method, which uses temperature to identify whether a sterilized article is sterilized, not only can assist the user to accurately identify whether a article is sterilized, but also when the sterilized article is used, the temperature can give a comfortable experience.

As a preferred embodiment, the sterilization container is preset with two constant temperatures of 35 degrees Celsius and 38 degrees Celsius. In the identification method, the sterilization container acquires an ambient temperature and adaptively selects an appropriate constant temperature according to the ambient temperature. Specifically, when the ambient temperature is lower than 15 degrees Celsius, a constant temperature of 35 degrees Celsius is selected, and when the ambient temperature is equal to or higher than 15 degrees Celsius, a constant temperature of 38 degrees Celsius is selected. The use of a variety of constant temperature temperatures corresponding to different ambient temperatures, while maintaining the above advantages, can effectively save energy. It should be noted that the constant temperature is not limited to two types, and is not limited to the specific temperature specified above, and may be flexibly set as actually needed according to the setting principle of the constant temperature in the present invention.

Further, in the identification method, the method for obtaining the ambient temperature of the sterilization container is to use the intracavity temperature estimation of the article storage cavity. In this way, it is not necessary to additionally install a temperature sensor to detect the ambient temperature, thereby simplifying the circuit and reducing the cost. The calculation method can obtain the ambient temperature by using the "intracavity temperature-ambient temperature correspondence table" obtained by experiments in advance, or can perform correlation calculation, for example, using the cavity temperature minus a constant to obtain the ambient temperature. The specific constants are obtained based on prior experimental statistics.

The intracavity temperature referred to herein may be the intracavity temperature at any time during the drying process of the sterilized article before and after sterilization.

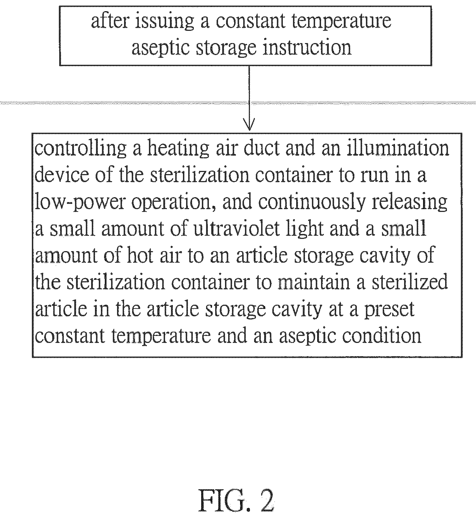

Referring to FIG. 2, the constant temperature aseptic storage method is applied to a sterilization container, and the constant temperature aseptic storage method includes the following steps: after issuing a constant temperature aseptic storage instruction, the sterilization container performing the following steps: controlling an illumination device and a heating air duct of the sterilization container to run in a low-power operation, and continuously releasing a small amount of ultraviolet light and a small amount of hot air to an article storage cavity of the sterilization container to maintain a sterilized article in the article storage cavity at a preset constant temperature and an aseptic condition; wherein the constant temperature is defined as when the sterilized article is in contact with the human body, the human body feels warm and not uncomfortable.

The constant temperature aseptic storage instruction can be manually issued by the user through a control button on the sterilization container, or can be released by a remote control method, such as application software on a remote controller or a smart phone, or can be automatically issued by a program in the sterilization container after the end of the sterilization or drying process.

After the constant temperature aseptic storage method is applied to the sterilization container, the user can take out the sterilized article at any time, and the sterilized article has a warm feeling, which can give the user a comfortable experience, and especially in a cold environment, the experience is more significant. Moreover, the sterilized article is sterile at any time when the sterilized article is taken out. In addition, the method is operated by reducing the power of the original illumination device and the heating air duct of the sterilization container, without adding any new structure, and thus has the advantage of low cost. The method of reducing power operation can be realized by adjusting the pulse width and frequency of the driving signal. For the power adjustment method itself, it is a conventional technique and will not be described herein.

As a preferred embodiment, the sterilization container is preset with two constant temperatures of 35 degrees Celsius and 38 degrees Celsius. In the constant temperature aseptic storage method, the sterilization container acquires an ambient temperature and adaptively selects an appropriate constant temperature according to the ambient temperature. Specifically, when the ambient temperature is lower than 15 degrees Celsius, a constant temperature of 35 degrees Celsius is selected, and when the ambient temperature is equal to or higher than 15 degrees Celsius, a constant temperature of 38 degrees Celsius is selected. The use of a variety of constant temperature temperatures corresponding to different ambient temperatures, while maintaining the above advantages, can effectively save energy. It should be noted that the constant temperature is not limited to two types, and is not limited to the specific temperature specified above, and may be flexibly set as actually needed according to the setting principle of the constant temperature in the present invention.

Further, in the constant temperature aseptic storage method, the method for obtaining the ambient temperature of the sterilization container is to use the intracavity temperature estimation of the article storage cavity. In this way, it is not necessary to additionally install a temperature sensor to detect the ambient temperature, thereby simplifying the circuit and reducing the cost. And, the calculation method can obtain the ambient temperature by using the "intracavity temperature-ambient temperature correspondence table" obtained by experiments in advance, or can perform correlation calculation, for example, using the cavity temperature minus a constant to obtain the ambient temperature. The specific constants are obtained based on prior experimental statistics.

The intracavity temperature referred to herein may be the intracavity temperature at any time during the drying process of the sterilized article before and after sterilization.

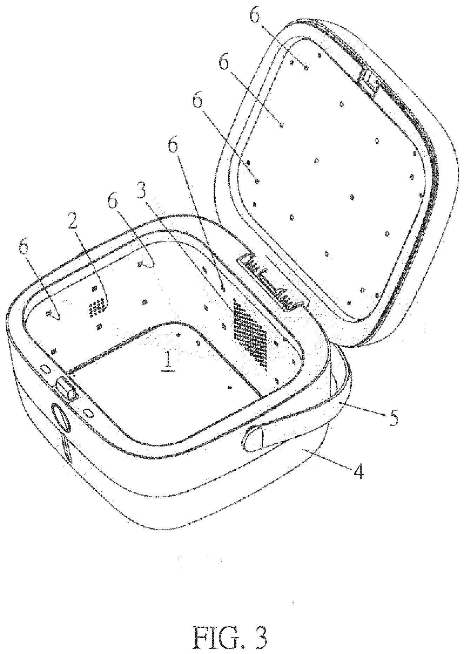

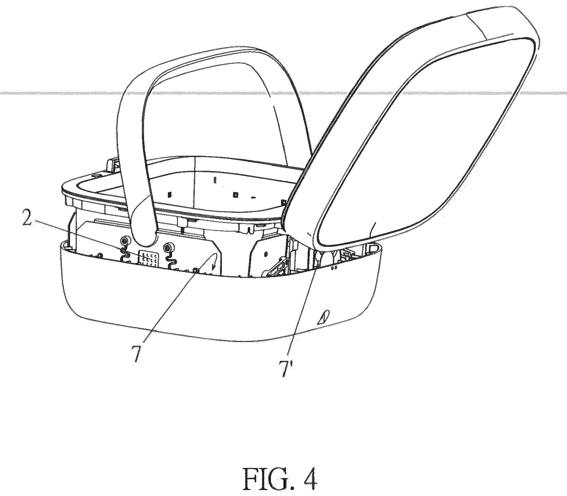

Referring to FIG. 3 and FIG. 4, the ultraviolet sterilization container includes: an article storage cavity 1 for accommodating an article to be sterilized, an illumination device 6 disposed on a cavity wall of the article storage cavity 1 for releasing ultraviolet light (sterilization medium) to the article storage cavity 1, and a heating air duct disposed on a cavity wall of the article storage cavity 1 for releasing hot air to the article storage cavity 1.

The heating air duct is an inner circulation type heating air duct, and the inner circulation type heating air duct includes: the article storage cavity 1; an air outlet 3 disposed on the cavity wall of the article storage cavity 1 and communicating with the article storage cavity 1; a return air outlet 2 disposed on the cavity wall of the article storage cavity 1 and communicating with the article storage cavity 1; an air duct 7 disposed inside the cavity wall of the article storage cavity 1 and communicating with the air outlet 3 and the return air outlet 2; and a fan 7' and an electric heating element 8 installed in the air duct 7 (as shown in FIG. 4). The inner circulation type heating air duct is formed in sequence from the article storage cavity 1, the air return outlet 2, the air duct 7, the fan 7', the electric heating element 8, the air outlet 3, and the article storage cavity 1. In this way, when drying, no air is introduced from the environment, and the circulating air is hot air and the air can be heated to a required temperature by using less energy, which can save energy consumed by drying.

The ultraviolet sterilization container further includes a microprocessor electrically coupled to the illumination device and the heating air duct, respectively, and a memory storing a computer readable program executable by the microprocessor; wherein the sterilization container performs the steps of the identification method and/or the steps of the constant temperature aseptic storage when the computer readable program is executed by the microprocessor. The memory may be an on-chip memory of the microprocessor or an external stand-alone memory.

In this way, the sterilization container has an identification function and/or a constant temperature aseptic storage function.

The sterilization container is a portable UV sterilization container that can be used not only at home but also for use in the office or when going out. The sterilization container body 4 is provided with a handle 5.

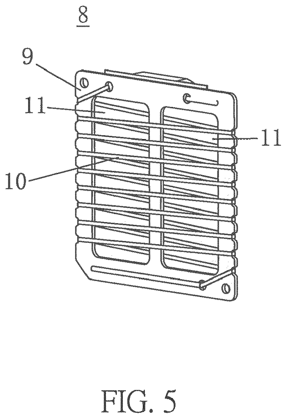

Further, as shown in FIG. 5, the electric heating element 8 of the heating air duct comprises a resistance wire 10 and a frame 9 having a hollow shape and a hollow portion 11, wherein the resistance wire 10 is wound around the frame 9 and a mesh structure is formed in the hollow portion 11. With such an electric heating element, it can be easily fixed to the end of the fan 7', and the installation of the electric heating element is very convenient. Moreover, air can pass through the mesh structure, which can effectively increase the contact area of the air and the electric heating element, achieving rapid and efficient heating of the flowing air.

The present invention has been described in detail with reference to the preferred embodiments thereof, and the detailed description is not to be construed as limiting the scope of the invention. Various refinements, equivalent transformations, and the like performed by the above-described embodiments under the present invention should be included in the scope of the present invention.

## Claims

1. An identification method applied to a sterilization container for identifying whether a sterilized article is sterilized, wherein the identification method comprises the following steps:
controlling a heating air duct of the sterilization container to run in a low-power operation after a sterilization is finished; and
continuously releasing a small amount of hot air to an article storage cavity of the sterilization container to maintain the sterilized article in the article storage cavity at a preset constant temperature;
wherein the constant temperature is defined as when the sterilized article is in contact with a human body, the human body feels warm and not uncomfortable.

2. The identification method according to claim 1, wherein the sterilization container is preset with a plurality of different constant temperatures; and in the identification method, the sterilization container acquires an ambient temperature and adaptively selects an appropriate constant temperature according to the ambient temperature.

3. The identification method according to claim 1, wherein in the identification method, the method for obtaining the ambient temperature of the sterilization container comprises using the intracavity temperature estimation of the article storage cavity.

4. A constant temperature aseptic storage method for a sterilization container, comprising: after issuing a constant temperature aseptic storage instruction, the sterilization container performing the following steps:
controlling a heating air duct and an illumination device of the sterilization container to run in a low-power operation; and
continuously releasing a small amount of ultraviolet light and a small amount of hot air to an article storage cavity of the sterilization container to maintain a sterilized article in the article storage cavity at a preset constant temperature and an aseptic condition;
wherein the constant temperature is defined as when the sterilized article is in contact with a human body, the human body feels warm and not uncomfortable.

5. The constant temperature aseptic storage method according to claim 4, wherein the sterilization container is preset with a plurality of different constant temperatures, and in the constant temperature aseptic storage method, the sterilization container acquires an ambient temperature and adaptively selects an appropriate constant temperature according to the ambient temperature.

6. The constant temperature aseptic storage method according to claim 4, wherein the method for obtaining the ambient temperature of the sterilization container comprises using the intracavity temperature estimation of the article storage cavity.

7. A sterilization container, comprising:
an article storage cavity configured to accommodate a sterilized article;
an illumination device configured to release a sterilization medium to the article storage cavity;
a heating air duct configured to release hot air to the article storage cavity;
a microprocessor electrically coupled to the illumination device and the heating air duct, respectively; and
a memory storing a computer readable program executable by the microprocessor;
wherein the sterilization container performs the steps of the identification method according to claim 1 when the computer readable program is executed by the microprocessor.

8. The sterilization container according to claim 7, wherein the heating air duct is an inner circulation type heating air duct and an electric heating element of the heating air duct comprises a resistance wire and a frame having a sheet shape and a hollow portion; and wherein the resistance wire is wound around the frame and a mesh structure is formed in the hollow portion.

9. The sterilization container according to claim 7, wherein the sterilization container is a portable ultraviolet sterilization container.

10. A sterilization container, comprising:
an article storage cavity configured to accommodate a sterilized article;
an illumination device configured to release a sterilization medium to the article storage cavity;
a heating air duct configured to release hot air to the article storage cavity;
a microprocessor electrically coupled to the illumination device and the heating air duct, respectively; and
a memory storing a computer readable program executable by the microprocessor;
wherein the sterilization container performs the steps of the constant temperature aseptic storage method according to claim 4 when the computer readable program is executed by the microprocessor.

11. The sterilization container according to claim 10, wherein the heating air duct is an inner circulation type heating air duct and an electric heating element of the heating air duct comprises a resistance wire and a frame having a sheet shape and a hollow portion; and wherein the resistance wire is wound around the frame and a mesh structure is formed in the hollow portion.

12. The sterilization container according to claim 10, wherein the sterilization container is a portable ultraviolet sterilization container.

13. A sterilization container, comprising:
an article storage cavity configured to accommodate a sterilized article;
an illumination device configured to release a sterilization medium to the article storage cavity;
a heating air duct configured to release hot air to the article storage cavity;
a microprocessor electrically coupled to the illumination device and the heating air duct, respectively; and
a memory storing a computer readable program executable by the microprocessor;
wherein when the computer readable program is executed by the microprocessor, the sterilization container performs the steps of the identification method according to claim 1 and the step of the constant temperature aseptic storage method according to claim 4.

14. The sterilization container according to claim 13, wherein the heating air duct is an inner circulation type heating air duct and an electric heating element of the heating air duct comprises a resistance wire and a frame having a sheet shape and a hollow portion; and wherein the resistance wire is wound around the frame and a mesh structure is formed in the hollow portion.

15. The sterilization container according to claim 13, wherein the sterilization container is a portable ultraviolet sterilization container.
